# EUROPEAN PATENT APPLICATION

(11) **EP 1 611 887 A2**
(43) Date of publication of application: **04.01.2006**
(21) Application number: 05291246.6
(22) Date of filing: 09.06.2005
(51) Int. Cl.: A61K 31/37, C12Q 1/26, C12N 9/04

(54) **Modulation of peroxisome proliferator-activated receptors**

(30) Priority: 10.06.2004 US 651469 P; 22.03.2005 US 664473 P
(71) Applicant: Abgenomics Corporation, Taipei, Taiwan 114 (TW)
(72) Inventor: Lin, Rong-Hwa, Da-an District Taipei Taiwan 106 (TW); Chuang, Lee-Ming, Xindian City Taipei Country Taiwan 231 (TW)
(74) Representative: Nargolwalla, Cyra

(57) **Abstract**

A method of treating a peroxisome proliferators-activated receptors related disease by administering to a subject in need thereof an effective amount of a modulator of 15-keto prostaglandin-Δ¹³-reductase. Also disclosed are methods of identifying a compound for inhibiting activity of the reductase and of lowering blood glucose levels by administering to a subject an effective amount of a reductase inhibitor.

### Annex to the application documents - subsequently filed sequences listing

SEQUENCE LISTING

<110> Lin, Rong-Hwa
      Chuang, Lee-Ming

<120> MODULATION OF PEROXISOME PROLIFERATOR-ACTIVATED RECEPTORS

<130> 13062-013001

<140> US 11/147,711
<141> 2005-06-08

<150> US 60/651,469
<151> 2004-06-10

<150> US 60/664,473
<151> 2005-03-22

<160> 8

<170> FastSEQ for Windows Version 4.0

<210> 1
<211> 351
<212> PRT
<213> Mus musculus

<400> 1
Met Ile Ile Gln Arg Val Val Leu Asn Ser Arg Pro Gly Lys Asn Gly
 1               5                  10                  15
Asn Pro Val Ala Glu Asn Phe Arg Val Glu Glu Phe Ser Leu Pro Asp
            20                  25                  30
Ala Leu Asn Glu Gly Gln Val Gln Val Arg Thr Leu Tyr Leu Ser Val
        35                  40                  45
Asp Pro Tyr Met Arg Cys Lys Met Asn Glu Asp Thr Gly Thr Asp Tyr
    50                  55                  60
Leu Ala Pro Trp Gln Leu Ala Gln Val Ala Asp Gly Gly Gly Ile Gly
65                  70                  75                  80
Val Val Glu Glu Ser Lys His Gln Lys Leu Thr Lys Gly Asp Phe Val
                85                  90                  95
Thr Ser Phe Tyr Trp Pro Trp Gln Thr Lys Ala Ile Leu Asp Gly Asn
            100                 105                 110
Gly Leu Glu Lys Val Asp Pro Gln Leu Val Asp Gly His Leu Ser Tyr
        115                 120                 125
Phe Leu Gly Ala Ile Gly Met Pro Gly Leu Thr Ser Leu Ile Gly Val
    130                 135                 140
Gln Glu Lys Gly His Ile Ser Ala Gly Ser Asn Gln Thr Met Val Val
145                 150                 155                 160
Ser Gly Ala Ala Gly Ala Cys Gly Ser Leu Ala Gly Gln Ile Gly His
                165                 170                 175
Leu Leu Gly Cys Ser Arg Val Val Gly Ile Cys Gly Thr Gln Glu Lys
            180                 185                 190
Cys Leu Phe Leu Thr Ser Glu Leu Gly Phe Asp Ala Ala Val Asn Tyr
        195                 200                 205
Lys Thr Gly Asn Val Ala Glu Gln Leu Arg Glu Ala Cys Pro Gly Gly

210                 215                 220
Val Asp Val Tyr Phe Asp Asn Val Gly Gly Asp Ile Ser Asn Ala Val
225                 230                 235                 240
Ile Ser Gln Met Asn Glu Asn Ser His Ile Ile Leu Cys Gly Gln Ile
                245                 250                 255
Ser Gln Tyr Ser Asn Asp Val Pro Tyr Pro Pro Pro Leu Pro Pro Ala
                260                 265                 270
Val Glu Ala Ile Arg Lys Glu Arg Asn Ile Thr Arg Glu Arg Phe Thr
        275                 280                 285
Val Leu Asn Tyr Lys Asp Lys Phe Glu Pro Gly Ile Leu Gln Leu Ser
    290                 295                 300
Gln Trp Phe Lys Glu Gly Lys Leu Lys Val Lys Glu Thr Met Ala Lys
305                 310                 315                 320
Gly Leu Glu Asn Met Gly Val Ala Phe Gln Ser Met Met Thr Gly Gly
                325                 330                 335
Asn Val Gly Lys Gln Ile Val Cys Ile Ser Glu Asp Ser Ser Leu
            340                 345                 350

<210> 2
<211> 1056
<212> DNA
<213> Mus musculus

<220>
<221> CDS
<222> (1)...(1053)

<400> 2
atg atc ata caa aga gtg gta ttg aat tcc cga cct ggg aaa aat gga      48
Met Ile Ile Gln Arg Val Val Leu Asn Ser Arg Pro Gly Lys Asn Gly
 1               5                  10                  15

aat cca gtc gca gag aac ttc agg gtg gaa gag ttc agt tta ccg gat      96
Asn Pro Val Ala Glu Asn Phe Arg Val Glu Glu Phe Ser Leu Pro Asp
            20                  25                  30

gct ctc aat gaa ggt caa gtt caa gtg agg act ctt tat ctc tcg gtg     144
Ala Leu Asn Glu Gly Gln Val Gln Val Arg Thr Leu Tyr Leu Ser Val
        35                  40                  45

gat cct tac atg cgc tgt aag atg aac gag gac act ggc act gac tac     192
Asp Pro Tyr Met Arg Cys Lys Met Asn Glu Asp Thr Gly Thr Asp Tyr
    50                  55                  60

ttg gca ccg tgg cag ctg gcg cag gtg gct gat ggt gga gga att gga     240
Leu Ala Pro Trp Gln Leu Ala Gln Val Ala Asp Gly Gly Gly Ile Gly
65                  70                  75                  80

gtt gta gag gag agc aag cac cag aag ttg act aaa ggc gat ttt gtg     288
Val Val Glu Glu Ser Lys His Gln Lys Leu Thr Lys Gly Asp Phe Val
                85                  90                  95

act tcg ttt tac tgg ccc tgg caa act aag gca att cta gat ggg aat     336
Thr Ser Phe Tyr Trp Pro Trp Gln Thr Lys Ala Ile Leu Asp Gly Asn
            100                 105                 110

ggc ctt gaa aag gta gac cca caa ctt gta gat gga cac ctt tca tat 384
Gly Leu Glu Lys Val Asp Pro Gln Leu Val Asp Gly His Leu Ser Tyr
        115                 120                 125

ttt ctt ggg gct ata ggt atg cct ggc ttg act tcc ttg att ggg gta 432
Phe Leu Gly Ala Ile Gly Met Pro Gly Leu Thr Ser Leu Ile Gly Val
    130                 135                 140

cag gag aaa ggc cat ata tct gct gga tct aat cag aca atg gtt gtc 480
Gln Glu Lys Gly His Ile Ser Ala Gly Ser Asn Gln Thr Met Val Val
145                 150                 155                 160

agt gga gca gca ggc gcc tgt gga tct ttg gct ggg cag att ggc cac 528
Ser Gly Ala Ala Gly Ala Cys Gly Ser Leu Ala Gly Gln Ile Gly His
                165                 170                 175

ctg ctt ggc tgt tcc aga gtg gtg gga att tgt gga acg cag gag aaa 576
Leu Leu Gly Cys Ser Arg Val Val Gly Ile Cys Gly Thr Gln Glu Lys
            180                 185                 190

tgt ctc ttt ttg acc tca gag ctg ggg ttt gat gct gca gtt aat tac 624
Cys Leu Phe Leu Thr Ser Glu Leu Gly Phe Asp Ala Ala Val Asn Tyr
        195                 200                 205

aaa aca ggg aat gtg gca gag cag ctg cga gaa gcg tgc ccg ggc gga 672
Lys Thr Gly Asn Val Ala Glu Gln Leu Arg Glu Ala Cys Pro Gly Gly
    210                 215                 220

gtg gat gtc tac ttt gac aat gtt gga ggt gac atc agc aac gcg gtg 720
Val Asp Val Tyr Phe Asp Asn Val Gly Gly Asp Ile Ser Asn Ala Val
225                 230                 235                 240

ata agt cag atg aat gag aac agc cac atc atc ctg tgt ggt cag att 768
Ile Ser Gln Met Asn Glu Asn Ser His Ile Ile Leu Cys Gly Gln Ile
                245                 250                 255

tct cag tac agt aac gat gtg ccc tac cct cct cca ctg ccc cct gca 816
Ser Gln Tyr Ser Asn Asp Val Pro Tyr Pro Pro Pro Leu Pro Pro Ala
            260                 265                 270

gta gaa gcc atc cgg aag gaa cga aac atc aca aga gag aga ttt acg 864
Val Glu Ala Ile Arg Lys Glu Arg Asn Ile Thr Arg Glu Arg Phe Thr
        275                 280                 285

gta tta aat tat aaa gat aaa ttt gag cct gga att cta cag ctg agt 912
Val Leu Asn Tyr Lys Asp Lys Phe Glu Pro Gly Ile Leu Gln Leu Ser
    290                 295                 300

cag tgg ttt aaa gaa gga aag cta aag gtc aag gag acc atg gca aag 960
Gln Trp Phe Lys Glu Gly Lys Leu Lys Val Lys Glu Thr Met Ala Lys
305                 310                 315                 320

ggc ttg gaa aac atg gga gtt gca ttc cag tcc atg atg aca ggg ggc 1008
Gly Leu Glu Asn Met Gly Val Ala Phe Gln Ser Met Met Thr Gly Gly
                325                 330                 335

aac gta ggg aaa cag atc gtc tgc att tca gaa gat tct tct ctg 1053

Asn Val Gly Lys Gln Ile Val Cys Ile Ser Glu Asp Ser Ser Leu
            340                 345                 350

tag 1056

<210> 3
<211> 20
<212> DNA
<213> Artificial Sequence

<220>
<223> Primer

<400> 3
cggtatagct tgggacgcta 20

<210> 4
<211> 22
<212> DNA
<213> Artificial Sequence

<220>
<223> Primer

<400> 4
tgcatgttaa gaatctttgt gg 22

<210> 5
<211> 26
<212> DNA
<213> Artificial Sequence

<220>
<223> Primer

<400> 5
aactgaagct tcaagtgatg atcata 26

<210> 6
<211> 22
<212> DNA
<213> Artificial Sequence

<220>
<223> Primer

<400> 6
agctctccca tatggtcgac ct 22

<210> 7
<211> 19
<212> RNA
<213> Artificial Sequence

<220>
<223> Synthetically generated siRNA

<400> 7
gaguucaguu uaccggaug                                                    19

<210> 8
<211> 19
<212> RNA
<213> Artificial Sequence

<220>
<223> Synthetically generated siRNA

<400> 8
guucaaguga ggacucuuu                                                    19

## Description

### CROSS REFERENCE TO RELATED APPLICATIONS

This application claims priority to U.S. Provisional Application Serial No. 60/664,473, filed on March 22, 2005, which in turn claims priority to U.S. Provisional Application Serial No. 60/651,469, filed as a U.S. Utility Application on June 10, 2004. The contents of both provisional applications are incorporated herein by reference in their entirety.

### BACKGROUND

Peroxisome proliferator-activated receptors (PPARs) belong to a family of nuclear receptors that regulate lipid and glucose metabolism. Three mammalian PPARs have been identified, i.e., PPAR-alpha, PPAR-gamma, and PPAR-delta. Upon activation by either dietary fatty acids or their metabolic derivatives, PPARs trigger a cascade of transcriptional events leading to altered lipid and glucose metabolism. For example, upon activation, PPAR-gamma, highly expressed in adipose tissues, promotes glucose uptake and lowers blood glucose levels.

Given their roles in lipid and glucose metabolism, PPARs are promising therapeutic targets of diseases, e.g., type II diabetes, obesity, dyslipidemia, coronary heart disease, inflammatory disease, and cancer. A synthetic PPAR-gamma agonist, i.e., AVANDIA, has been used to treat type II diabetes. Another synthetic PPAR-alpha agonist, i.e., Fibrates, has been used to treat dyslipidemia. See Lehmann, et al., J Biol Chem, (1995) 270:12953-12956; Fruchart, et al., Curr. Opin. Lipdol. (1999) 10:245-257. However, most PPAR therapeutics have limited efficacy and significant side effects.

There is a need to develop more effective drugs for controlling lipid and glucose metabolism via modulation of PPAR activity.

### SUMMARY

This invention relates to methods of treating PPAR related diseases via modulation of PPAR activity in a subject.

In one aspect, this invention features an isolated polypeptide that reduces 15-keto prostaglandin but not leukotriene B4. Prostaglandin (PG) is a class of physiological mediators characterized by a central ring and side chains of varying degrees of unsaturation. Examples of 15-keto prostaglandin include but are not limited to 15-keto PGE₂, 15-keto PGE_{1,} 15-keto PGF_{2α}, and 15-keto PGF_{1α}. Leukotriene, another class of physiological mediators, differs in part from prostaglandin in not having a central ring.

In another aspect, this invention features an antibody that binds specifically to the polypeptide described above. The antibody, either polyclonal or monoclonal, may bind to a fragment of the polypeptide.

In still another aspect, this invention features a double-stranded ribonucleic acid (dsRNA), as well as a DNA vector encoding it, for inhibiting expression of a polypeptide with 15-keto prostaglandin-Δ¹³-reductase activity. 15-keto prostaglandin-Δ¹³-reductase refers to an enzyme that catalyzes the conversion of a 15-keto prostaglandin to 13,14-dihydro-15-keto prostaglandin by reducing the Δ¹³ double bond of the prostaglandin. Examples of 15-keto prostaglandin-Δ¹³-reductases include 15-keto prostaglandin-Δ¹³-reductase/leukotriene B4 12-hydroxydehydrogenase (PGR/LTB4DH) and zinc binding alcohol dehydrogenase 1 (PGR2/ZADH1). The dsRNA contains two strands of polyribonucleotide. The first strand is identical to 19 to 49 consecutive nucleotides of a nucleic acid that encodes 15-keto prostaglandin-Δ¹³-reductase. The second strand is complementary to the first strand. Preferably, at least one end of the dsRNA has an overhang of 1 to 4 nucleotides. The 15-keto prostaglandin-Δ¹³-reductase can be PGR/LTB4DH or PGR2/ZADH1.

This invention also covers a method of treating a PPAR related disease such as type II diabetes, obesity, dyslipidemia, coronary heart disease, inflammatory disease, and cancer. The method includes administering to a subject an effective amount of a 15-keto prostaglandin-Δ¹³-reductase modulator. A15-keto prostaglandin-Δ¹³-reductase modulator refers to a molecule or a complex of molecules that affects activity or expression of 15-keto prostaglandin-Δ¹³-reductase. A modulator can be a 15-keto prostaglandin. It can also be an inhibitor that suppresses either activity or expression of 15-keto prostaglandin-Δ¹³-reductase, e.g., the above-described dsRNA, coumarin, flavonoid, or hydroxychalcone.

Also within the scope of this invention is a method of lowering blood glucose levels in a subject. The method includes administering to the subject an effective amount of a 15-keto prostaglandin-Δ¹³-reductase inhibitor, which includes a coumarin, flavonoid, or hydroxychalcone. The inhibitor can suppress either activity or expression of 15-keto prostaglandin-Δ¹³-reductase.

This invention further features a method of identifying a compound that inhibits 15-keto prostaglandin-Δ¹³-reductase activity. Inhibition refers to suppression of either activity or expression of 15-keto prostaglandin-Δ¹³-reductase. The method includes providing a system containing 15-keto prostaglandin-Δ¹³-reductase, contacting a compound with the system, determining the 15-keto prostaglandin-Δ¹³-reductase activity, and comparing the activity with that obtained in the same manner except that the compound is absent. In one embodiment, the system is a cell containing a gene that expresses 15-keto prostaglandin-Δ¹³-reductase and the activity is determined by measuring expression activity of the gene. In another embodiment, the system is a cell-free solution that contains 15-keto prostaglandin-Δ¹³-reductase and the activity is determined by measuring enzymatic 15-keto prostaglandin-Δ¹³-reductase activity of the cell-free solution. The 15-keto prostaglandin-Δ¹³-reductase can be PGR/LTB4DH or PGR2/ZADH 1.

The details of one or more embodiments of the invention are set forth in the description below. Other features, objects, and advantages of the invention will be apparent from the description and from the claims.

### DETAILED DESCRIPTION

The present invention is based on the discovery of 15-keto prostaglandin-Δ¹³-reductase 2 (PGR-2), a member of the 15-keto prostaglandin-Δ¹³-reductase family. It was found unexpectedly that PPAR activity can be controlled by its substrates and inhibitors. These substrates and inhibitors are useful for treating PPAR related diseases, e.g., type II diabetes, obesity, dyslipidemia, coronary heart disease, inflammatory disease, and cancer.

Contemplated within the scope of this invention is an isolated polypeptide of SEQ ID NO: 1 or its functional equivalent that reduces 15-keto prostaglandin but not leukotriene B4. Shown below is its amino acid sequence (SEQ ID NO:1), as well as the encoding nucleotide sequence (i.e., SEQ ID NO:2).

1 - ATGATCATACAAAGAGTGGTATTGAATTCCCGACCTGGGAAAAATGGAAATCCAGTCGCA - 60
- M I I Q R V V L N S R P G K N G N P V A
61 - GAGAACTTCAGGGTGGAAGAGTTCAGTTTACCGGATGCTCTCAATGAAGGTCAAGTTCAA - 120
- E N F R V E E F S L P D A L N E G Q V Q
121 - GTGAGGACTCTTTATCTCTCGGTGGATCCTTACATGCGCTGTAAGATGAACGAGGACACT - 180
- V R T L Y L S V D P Y M R C K M N E D T
181 - GGCACTGACTACTTGGCACCGTGGCAGCTGGCGCAGGTGGCTGATGGTGGAGGAATTGGA - 240
- G T D Y L A P W Q L A Q V A D G G G I G
241 - GTTGTAGAGGAGAGCAAGCACCAGAAGTTGACTAAAGGCGATTTTGTGACTTCGTTTTAC - 300
- V V E E S K H Q K L T K G D F V T S F Y
301 - TGGCCCTGGCAAACTAAGGCAATTCTAGATGGGAATGGCCTTGAAAAGGTAGACCCACAA - 360
- W P W Q T K A I L D G N G L E K V D P Q
361 - CTTGTAGATGGACACCTTTCATATTTCTTGGGGCTATAGGTATGCCTGGCTTGACTTCC - 420
- L V D G H L S Y F L G A I G M P G L T S
421 - TTGATTGGGGTACAGGAGAAAGGCCATATATCTGCTGGATCTAATCAGACAATGGTTGTC - 480
- L I G V Q E K G H I S A G S N Q T M V V
481 - AGTGGAGCAGCAGGCGCCTGTGGATCTTTGGCTGGGCAGATTGGCCACCTGCTTGGCTGT - 540
- S G A A G A C G S L A G Q I G H L L G C
541 - TCCAGAGTGGTGGGAATTTGTGGAACGCAGGAGAAATGTCTCTTTTTGACCTCAGAGCTG - 600
- S R V V G I C G T Q E K C L F L T S E L
601 - GGGTTTGATGCTGCAGTTAATTACAAAACAGGGAATGTGGCAGAGCAGCTGCGAGAAGCG - 660
- G F D A A V N Y K T G N V A E Q L R E A
661 - TGCCCGGGCGGAGTGGATGTCTACTTTGACAATGTTGGAGGTGACATCAGCAACGCGGTG - 720
- C P G G V D V Y F D N V G G D I S N A V
721 - ATAAGTCAGATGAATGAGAACAGCCACATCATCCTGTGTGGTCAGATTTCTCAGTACAGT - 780
- I S Q M N E N S H I I L C G Q I S Q Y S
781 - AACGATGTGCCCTACCCTCCTCCACTGCCCCCTGCAGTAGAAGCCATCCGGAAGGAACGA - 840
- N D V P Y P P P L P P A V E A I R K E R
841 - AACATCACAAGAGAGAGATTTACGGTATTAAATTATAAAGATAAATTTGAGCCTGGAATT - 900
- N I T R E R F T V L N Y K D K F E P G I
901 - CTACAGCTGAGTCAGTGGTTTAAAGAAGGAAAGCTAAAGGTCAAGGAGACCATGGCAAAG - 960
- L Q L S Q W F K E G K L K V K E T M A K
961 - GGCTTGGAAAACATGGGAGTTGCATTCCAGTCCATGATGACAGGGGGCAACGTAGGGAAA - 1020
- G L E N M G V A F Q S M M T G G N V G K
1021 - CAGATCGTCTGCATTTCAGAAGATTCTTCTCTGTAG (SEQ ID NO:2) - 1056
- Q I V C I S E D S S L * (SEQ ID NO:1)

An isolated polypeptide refers to a polypeptide substantially free from naturally associated molecules, i.e., it is at least 75% (i.e., any number between 75% and 100%, inclusive) pure by dry weight. Purity can be measured by any appropriate standard method, for example, by column chromatography, polyacrylamide gel electrophoresis, or HPLC analysis. An isolated polypeptide of the invention can be purified from a natural source, produced by recombinant DNA techniques, or by chemical methods. The term "functional equivalent" refers to a variant of a polypeptide of SEQ ID NO: 1 that possesses the ability to reduce 15-keto prostaglandin but not leukotriene B4, e.g., a protein having one or more point mutations, insertions, deletions, truncations, or a combination thereof. In one embodiment, an isolated polypeptide of the invention or its functional equivalent contains a sequence that is at least 80% (e.g., 85%, 95%, or 100%, or any other number between 80% and 100%, inclusive) identical to SEQ ID NO: 1. It can be a fusion protein.

15-keto prostaglandin-Δ¹³-reductase activity refers to the enzymatic conversion of 15-keto prostaglandin to 13,14-dihydro-15-keto prostaglandin. The specific activity is determined as follows: 10 µg of a protein preparation to be assayed is incubated at 37°C in a reaction buffer containing 0.1M Tris-HCl (pH 7.4), 0.5 mM NADPH, and 0.57 mM 15-keto PGE₂. The reaction is conducted in the dark for 10 minutes at 37°C and terminated by adding 700 µl of a buffer containing 50 mM potassium hydrogen phthalate, pH 3.0, and 1% Tween 20. 200 µl of a color development reagent, which contains 790 µM indonitrotetrazolium chloride, 60 µM phenazene methosulfate, and 1% Tween 20, is used to oxidize any unreacted NADPH. Absorbance at 490 nm is measured using an ELISA plate reader. A standard curve is generated using reaction buffers containing serially diluted amounts of NADPH. A specific activity of at least 90 nmole/min.mg protein indicates that the polypeptide has 15-keto prostaglandin-Δ¹³-reductase activity.

The nucleotide sequence described above, i.e., SEQ ID NO:2, can be used to express the polypeptide of this invention. A nucleotide sequence refers to a DNA molecule (e.g., a cDNA or genomic DNA), an RNA molecule (e.g., an mRNA), or a DNA or RNA analog. A DNA or RNA analog can be synthesized from nucleotide analogs. The nucleic acid molecule can be single-stranded or double-stranded, but preferably double-stranded. For the purpose of protein expression, one can operatively linked the nucleic acid to suitable regulatory sequences to generate an expression vector. A vector refers to a nucleic acid molecule capable of transporting another nucleic acid to which it has been linked. The vector can be capable of autonomous replication or integrate into a host DNA. Examples of the vector include a plasmid, cosmid, or viral vector. The vector may include a nucleotide sequence in a form suitable for expression of the nucleic acid in a host cell. Preferably the vector includes one or more regulatory sequences operatively linked to the nucleic acid sequence to be expressed. A "regulatory sequence" includes promoters, enhancers, and other expression control elements (e.g., polyadenylation signals). Regulatory sequences include those that direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vector can be introduced into host cells to produce the polypeptide of this invention. Also within the scope of this invention is a host cell that contains the above-described nucleic acid. Examples include *E*. *coli* cells, Sf9 insect cells (e.g., using baculovirus expression vectors), yeast cells, or mammalian cells. See e.g., Goeddel, (1990) Gene Expression Technology: Methods in Enzymology 185, Academic Press, San Diego, CA. To produce a polypeptide of this invention, one can culture a host cell in a medium under conditions permitting expression of the polypeptide encoded by a nucleic acid of this invention, and purify the polypeptide from the cultured cell or the medium of the cell. Alternatively, the nucleotide sequence can be transcribed and translated in vitro, for example, using T7 promoter regulatory sequences and T7 polymerase.

The above-described polypeptide can be used to generate antibodies in animals. It is understood that the antibodies can also be generated from a fragment of the polypeptide. Methods of making monoclonal and polyclonal antibodies and fragments thereof in animals are known in the art. See, for example, Harlow and Lane, (1988) Antibodies: A Laboratory Manual, Cold Spring Harbor Laboratory, New York. The term "antibody" includes intact molecules as well as fragments thereof, such as Fab, F(ab')₂, Fv, scFv (single chain antibody), and dAb (domain antibody; Ward, et. al. (1989) Nature, 341, 544).

In general, a polypeptide of this invention can be coupled to a carrier protein, such as KLH, mixed with an adjuvant, and injected into a host animal. Antibodies produced in that animal can then be purified by peptide affinity chromatography. Commonly employed host animals include rabbits, mice, guinea pigs, and rats. Various adjuvants that can be used to increase the immunological response depend on the host species and include Freund's adjuvant (complete and incomplete), mineral gels such as aluminum hydroxide, surface active substances such as lysolecithin, pluronic polyols, polyanions, peptides, oil emulsions, keyhole limpet hemocyanin, and dinitrophenol. Useful human adjuvants include BCG (bacille Calmette-Guerin) and Corynebacterium parvum.

Polyclonal antibodies, heterogeneous populations of antibody molecules, are present in the sera of the immunized subjects. Monoclonal antibodies, homogeneous populations of antibodies to a polypeptide of this invention, can be prepared using standard hybridoma technology (see, for example, Kohler et al. (1975) Nature 256, 495; Kohler et al. (1976) Eur J Immunol 6, 511; Kohler et al. (1976) Eur J Immunol 6, 292; and Hammerling et al. (1981) Monoclonal Antibodies and T Cell Hybridomas, Elsevier, N.Y.). In particular, monoclonal antibodies can be obtained by any technique that provides for the production of antibody molecules by continuous cell lines in culture such as described in Kohler et al. (1975) Nature 256, 495 and U.S. Patent No. 4,376,110; the human B-cell hybridoma technique (Kosbor et al. (1983) Immunol Today 4, 72; Cole et al. (1983) Proc. Natl. Acad Sci. USA 80, 2026, and the EBV-hybridoma technique (Cole et al. (1983) Monoclonal Antibodies and Cancer Therapy, Alan R. Liss, Inc., pp. 77-96). Such antibodies can be of any immunoglobulin class including IgG, IgM, IgE, IgA, IgD, and any subclass thereof. The hybridoma producing the monoclonal antibodies of the invention may be cultivated in vitro or in vivo. The ability to produce high titers of monoclonal antibodies in vivo makes it a particularly useful method of production.

In addition, techniques developed for the production of "chimeric antibodies" can be used. See, e.g., Morrison et al. (1984) Proc. Natl. Acad. Sci. USA 81, 6851; Neuberger et al. (1984) Nature 312, 604; and Takeda et al. (1984) Nature 314:452. A chimeric antibody is a molecule in which different portions are derived from different animal species, such as those having a variable region derived from a murine monoclonal antibody and a human immunoglobulin constant region. Alternatively, techniques described for the production of single chain antibodies (U.S. Patent Nos. 4,946,778 and 4,704,692) can be adapted to produce a phage library of single chain Fv antibodies. Single chain antibodies are formed by linking the heavy and light chain fragments of the Fv region via an amino acid bridge. Moreover, antibody fragments can be generated by known techniques. For example, such fragments include, but are not limited to, F(ab')₂ fragments that can be produced by pepsin digestion of an antibody molecule, and Fab fragments that can be generated by reducing the disulfide bridges of F(ab')₂ fragments. Antibodies can also be humanized by methods known in the art. For example, monoclonal antibodies with a desired binding specificity can be commercially humanized (Scotgene, Scotland; and Oxford Molecular, Palo Alto, Calif.). Fully human antibodies, such as those expressed in transgenic animals are also features of the invention (see, e.g., Green et al. (1994) Nature Genetics 7, 13; and U.S. Patent Nos. 5,545,806 and 5,569,825).

Also within the scope of this invention is a double-stranded ribonucleic acid (dsRNA). This dsRNA can be used to inhibit expression of 15-keto prostaglandin-Δ¹³-reductase. Thus, one can treat a PPAR related disease by administering the dsRNA to a subject. The term "dsRNA" refers to a double-stranded ribonucleic acid that silences gene expression via degradation of a targeted RNA sequence, a process known as RNA interference (RNAi). RNAi has been used to silence gene expression in a wide variety of animal models (including C. elegans, zebrafish, and mouse embryos) and in other biological systems (including explanted chick neural cells and mammalian cell culture). See WO99/32619, WO00/44914, WO00/44914, WO00/44895, WO00/63364, and WO01/36646 A1.

An RNA of this invention can be synthesized by techniques well known in the art. See, e.g., Caruthers et al., 1992, Methods in Enzymology 211, 3-19, Wincott et al., 1995, Nucleic Acids Res. 23, 2677-2684, Wincott et al., 1997, Methods Mol. Bio. 74, 59, Brennan et al., 1998, Biotechnol Bioeng., 61, 33-45, and Brennan, U.S. Patent No. 6,001,311. The RNA can also be transcribed from an expression vector and isolated using standard techniques. An RNA or vector of this invention can be delivered to target cells using method also well known in the art. See, e.g., Akhtar et al., 1992, Trends Cell Bio. 2, 139. For example, it can be introduced into cells using liposomes, hydrogels, cyclodextrins, biodegradable nanocapsules, or bioadhesive microspheres. Alternatively, the RNA or vector is locally delivered by direct injection or by use of an infusion pump. Other approaches include use of various transport and carrier systems, e.g., using conjugates and biodegradable polymers.

This invention also features a method of treating PPAR related diseases by modulating 15-keto prostaglandin-Δ¹³-reductase activity or expression. The term "treating" refers to administering one or more of the above-described 15-keto prostaglandin-Δ¹³-reductase modulators, i.e., 15-keto prostaglandin-Δ¹³-reductase substrates and inhibitors, to a subject who has a PPAR related disease, a symptom of such a disease, or a predisposition toward such a disease, with the purpose to confer a therapeutic effect, e.g., to cure, relieve, alter, affect, ameliorate, or prevent the PPAR related disease, the symptom of it, or the predisposition toward it. "An effective amount" refers to the amount that is required to confer a therapeutic effect on a treated subject. A substrate of 15-keto prostaglandin-Δ¹³-reductase includes 15-keto PGE₂, 15-keto PGE₁, 15-keto PGF_{2α}, 15-keto PGF_{1α}, and structural analogs thereof.

Examples of PPAR related diseases (or disorders or conditions) include, but are not limited to, type II diabetes, hyperglycemia, low glucose tolerance, Syndrome X, insulin resistance, obesity, lipid disorders, dyslipidemia, hyperlipidemia, hyperglycemia, hypertriglyceridemia, hypercholesterolemia, low HDL levels, high LDL levels, atherosclerosis (and its sequelae such as angina, claudication, heart attack, or stroke), vascular restenosis, irritable bowel syndrome, inflammatory diseases (e.g., inflammatory bowel disease, rheumatoid arthritis, Crohn's disease, ulcerative colitis, osteoarthritis, multiple sclerosis, asthma, vasculitis, ischemia/reperfusion injury, frostbite, or adult respiratory distress syndrome), pancreatitis, neurodegenerative disease, retinopathy, neoplastic conditions, cancers (e.g., prostate, gastric, breast, bladder, lung, or colon cancer, or adipose cell cancer such as liposarcoma), angiogenesis, Alzheimer's disease, skin disorders (e.g., acne, psoriasis, dermatitis, eczema, or keratosis), high blood pressure, ovarian hyperandrogenism, osteoporosis, and osteopenia.

To treat a PPAR related disease, a pharmaceutical composition containing a 15-keto prostaglandin-Δ¹³-reductase modulator and a pharmaceutically acceptable carrier can be administered to a subject in need thereof. It can be administered orally or by intravenous infusion, or injected or implanted subcutaneously, intramuscularly, intrathecally, intraperitoneally, intrarectally, intravaginally, intranasally, intragastrically, intratracheally, or intrapulmonarily.

The pharmaceutical composition can be a solution or suspension in a non-toxic acceptable diluent or solvent, such as a solution in 1,3-butanediol. Among the acceptable vehicles and solvents that can be employed are mannitol, water, Ringer's solution, and isotonic sodium chloride solution. In addition, fixed oils are conventionally employed as a solvent or suspending medium (e.g., synthetic mono- or diglycerides). Fatty acid, such as oleic acid and its glyceride derivatives are useful in the preparation of injectables, as are natural pharmaceutically acceptable oils, such as olive oil or castor oil, especially in their polyoxyethylated versions. These oil solutions or suspensions can also contain a long chain alcohol diluent or dispersant, carboxymethyl cellulose, or similar dispersing agents. Other commonly used surfactants such as Tweens or Spans or other similar emulsifying agents or bioavailability enhancers which are commonly used in the manufacture of pharmaceutically acceptable solid, liquid, or other dosage forms can also be used for the purpose of formulation.

The dosage required depends on the choice of the route of administration; the nature of the formulation; the nature of the subject's illness; the subject's size, weight, surface area, age, and sex; other drugs being administered; and the judgment of the attending physician. Suitable dosages may be in the range of 0.01-100.0 mg/kg. Wide variations in the needed dosage are to be expected in view of the variety of compositions available and the different efficiencies of various routes of administration. Variations in these dosage levels can be adjusted using standard empirical routines for optimization as is well understood in the art. Encapsulation of the composition in a suitable delivery vehicle (e.g., polymeric microparticles or implantable devices) may increase the efficiency of delivery, particularly for oral delivery.

The above-described pharmaceutical composition can be formulated into dosage forms for different administration routes utilizing conventional methods. For example, it can be formulated in a capsule, a gel seal, or a tablet for oral administration. Capsules can contain any standard pharmaceutically acceptable materials such as gelatin or cellulose. Tablets can be formulated in accordance with conventional procedures by compressing mixtures of the composition with a solid carrier and a lubricant. Examples of solid carriers include starch and sugar bentonite. The composition can also be administered in a form of a hard shell tablet or a capsule containing a binder, e.g., lactose or mannitol, a conventional filler, and a tableting agent. The pharmaceutical composition can be administered via the parenteral route. Examples of parenteral dosage forms include aqueous solutions, isotonic saline or 5% glucose of the active agent, or other well-known pharmaceutically acceptable excipient. Cyclodextrins, or other solubilizing agents well known to those familiar with the art, can be utilized as pharmaceutical excipients for delivery of the therapeutic agent.

The efficacy of the above-described pharmaceutical composition can be evaluated both in vitro and in vivo. Briefly, the pharmaceutical composition can be tested for its ability to inhibit 15-keto prostaglandin-Δ¹³-reductase activity or expression in vitro. For in vivo studies, the pharmaceutical composition can be injected into an animal (e.g., a mouse model) and its therapeutic effects are then accessed. Based on the results, an appropriate dosage range and administration route can be determined.

The invention further features a method of identifying a compound for inhibiting 15-keto prostaglandin-Δ¹³-reductase activity or expression. The compound can be designed, e.g., using computer modeling programs, according to the three-dimensional conformation of the polypeptide, and synthesized using methods known in the art. It can also be identified by library screening, or obtained using any of the numerous approaches in combinatorial library methods known in the art. Suitable libraries include: peptide libraries, peptoid libraries (libraries of molecules having the functionalities of peptides, but with a novel, non-peptide backbone that is resistant to enzymatic degradation), spatially addressable parallel solid phase or solution phase libraries, synthetic libraries obtained by deconvolution or affinity chromatography selection, the "one-bead one-compound" libraries, and antibody libraries. See, e.g., Zuckermann et al. (1994) J. Med. Chem. 37, 2678-85; Lam (1997) Anticancer Drug Des. 12, 145; Lam et al. (1991) Nature 354, 82; Houghten et al. (1991) Nature 354, 84; and Songyang et al. (1993) Cell 72, 767. Examples of methods for the synthesis of molecular libraries can be found in the art, for example, in: DeWitt et al. (1993) Proc. Natl. Acad. Sci. USA 90, 6909; Erb et al. (1994) Proc. Natl. Acad. Sci. USA 91, 11422; Zuckermann et al. (1994) J. Med. Chem. 37, 2678; Cho et al. (1993) Science 261, 1303; Carrell et al. (1994) Angew. Chem. Int. Ed. Engl. 33, 2059; Carell et al. (1994) Angew. Chem. Int. Ed. Engl. 33, 2061; and Gallop et al. (1994) J. Med. Chem. 37,1233. Libraries of compounds may be presented in solution (e.g., Houghten (1992) Biotechniques 13, 412-421), or on beads (Lam (1991) Nature 354, 82-84), chips (Fodor (1993) Nature 364, 555-556), bacteria (U.S. Patent No. 5,223,409), spores (U.S. Patent No. 5,223,409), plasmids (Cull et al. (1992) Proc. Natl. Acad. Sci. USA 89, 1865-1869), or phages (Scott and Smith (1990) Science 249, 386-390; Devlin (1990) Science 249, 404-406; Cwirla et al. (1990) Proc. Natl. Acad. Sci. USA 87, 6378-6382; Felici (1991) J. Mol. Biol. 222, 301-310; and U.S. Patent No. 5,223,409).

The specific examples below are to be construed as merely illustrative, and not limitative of the remainder of the disclosure in any way whatsoever. Without further elaboration, it is believed that one skilled in the art can, based on the description herein, utilize the present invention to its fullest extent. All publications cited herein are hereby incorporated by reference in their entirety.

### Identification of a novel 15-keto prostaglandin-Δ¹³-reductase

To identify genes up-regulated during adipogenesis, mRNA differential display analysis was performed using mouse 3T3-L1 cells. To induce adipogenesis, 3T3-L1 cells were treated with 1 µM dexamethasone and allowed to grow for 10 days at 37°C. A 199-nucleotide fragment was isolated and found to be highly expressed in 3T3-L1 cells harvested on the 10^{th} day after induction. The sequence of this fragment was determined to be identical to a segment of two GenBank entries, i.e., AK021033 and AK020666.

The full-length cDNA sequence corresponding to the coding region of the gene was referred to as mouse PGR-2. This sequence was isolated and cloned from 3T3-L1 adipocytes as follows. PGR-2 cDNA was PCR-amplified and ligated into a pGEM-T easy vector (Promega) by T4 DNA ligase (Promega). The sequences of forward and reverse primers for amplifying PGR-2 cDNA were 5'-CGG TAT AGC TTG GGA CGC TA-3' (SEQ ID NO:3) and 5'-TGC ATG TTA AGA ATC TTT GTG G-3' (SEQ ID NO:4), respectively. The resulting construct (pTE-PGR-2) was then sequenced by T7 and SP6 polymerases. The coding region of PGR-2 open reading frame was then subcloned to the expression vector pCMV-Tag2B (Stratagene). For constructing pFLAG-PGR-2, a PCR reaction was conducted to generate a *Hind*III-*Sal*I fragment of PGR-2 using pTE-PGR-2 as a template and two oligonucleotides as primers, 5'-AAC TGA AGC TTC AAG TGA TGA TCA TA-3' (SEQ ID NO:5) and 5'-AGC TCT CCC ATA TGG TCG ACC T-3' (SEQ ID NO:6). The PCR product thus obtained was then introduced into the *Hind*III-*Sal*I sites of pCMV-Tag2B, yielding a fused construct of pFLAG/PGR-2. Finally, the pGEX-PGR-2 construct was prepared by ligating the *Hind*III-*Xho*I fragment of pFLAG/PGR-2 into a pGEX-4T-3 vector restricted with *Sma*I and *Xho*I (Pharmacia).

The deduced amino acid sequence of mouse PGR-2, i.e., SEQ ID NO:1, is shown above. The mouse PGR-2 was found to be homologous to two proteins: (1) human ZADH1 (GenBank accession no.: NM152444) with ~92% homology, and (2) PGR/LTB4DH or PGR-1 with ~54% homology.

PGR-2 expression increased during adipogenesis in 3T3-L1 cells. The maximal expression was observed at day 6 after induction of adipogenesis. At this time point, lipid droplets were observed to accumulate extensively in the adipocytes. The tissue distribution of PGR-2 was determined. It was highly expressed in adipose tissue. The amount of PGR-2 mRNA in omental fat was significantly higher in both homozygous and heterozygous db/db mice than in wild type mice.

Mouse PGR-2 was recombinantly expressed in E.coli as a GST fusion protein following standard procedures. The recombinant PGR-2 protein thus obtained was used to determine substrate specificity and enzymatic kinetics.

Enzymatic activity was determined as follows. 10 µg of the recombinant mouse PGR-2 protein was incubated at 37°C in a reaction buffer containing 0.1 M Tris-HCl (pH7.4), 0.5 mM NADPH, and 0.57 mM 15-keto PGE₂. The reaction was conducted at 37°C in the dark for 10 minutes and terminated by adding 700 µl of a buffer which contained 50 mM potassium hydrogen phthalate, pH 3.0, and 1% Tween 20. 200 µl of a color development reagent, which contained 790 µM indonitrotetrazolium chloride, 60 µM phenazene methosulfate, and 1% Tween 20, was added to oxidize any unreacted NADPH. Absorbance at 490 nm was measured by an ELISA plate reader. A standard curve was generated using reaction buffers containing serially diluted amounts of NADPH.

Substrate specificity of PGR-2 was determined using the just-described procedure, except that 15-keto PGE₂ was replaced with each of six prostaglandin substrates, each of three downstream metabolites, or leukotriene B4. 15-keto PGE₁, 15-keto PGF_{1α,} and 15-keto PGF_{2α} reacted specifically with PGR-2. By contrast, no specific activity was detected from 6-keto PGF1_{α}, PGF_{2β}, 11b-PGF_{2α}, 13,14-dihydro-15-keto PGF_{2α}, 13,14-dihydro-15-keto PGD₂, 13,14-dihydro-15-keto PGE₂, and leukotriene B4.

Kinetics studies indicated that PGR-2 catalyzed conversion of 15-keto PGE₂ into 13,14-dihydro-15-keto PGE₂ most efficiently, followed by 15-keto PGF_{1α}, 15-keto PGE₁, and 15-keto PGF_{2α}. Unlike PGR/LTB4DH, PGR-2 used NADPH as a cofactor much more efficiently than NADH.

The protein expression level of PGR-2 was up-regulated during adipogenesis in 3T3-L1 cells. The maximal PGR-2 protein level was detected in fully differentiated adipocytes. PPAR-gamma was induced markedly at an earlier stage of adipogenesis. Low PGR-2 expression was localized in the nuclei in pre-adipocytes. Higher PGR-2 expression was distributed in the cytoplasm of the differentiated adipocytes.

Also investigated was the effect of PGR-2 expression on modulating PPAR-gamma transcription in human Hep3B cells, which expressed endogenous human PPAR-alpha and -gamma. Over-expression of PGR-2 in Hep3B cells was found to suppress PPAR-mediated transcriptional activation. The transcriptional activation was also suppressed even after Hep3B cells were stimulated by a PPAR-gamma agonist, i.e., BRL49653. Similar results were obtained from 3T3-L1 cells.

### Prostaglandin

The effect of prostaglandin on PPAR-gamma activity in adipocytes was investigated. After treatment with a medium that induces cell differentiation, 3T3-L1 cells were treated from day 2 to 4 during adipogenesis with 14 µM 15-keto PGE₂, 13,14-dihydro-15-keto PGE₂, 15-keto PGF_{2α}, 13,14-dihydro-15-keto PGF_{2α}, or 4.5 µM of BRL49653, a PPAR-gamma agonist. See Forman et al., Cell (1995) 83:803-812. At day 6, aggregates of lipid droplets were stained with oil-red O for observation. 15-keto PGE₂ effectively enhanced adipogenesis at a level similar to BRL49653. After being induced to differentiate for two days, the 3T3-L 1 cells were transfected with a reporter gene. Both 15-keto PGE₂ and 15-keto PGF_{2α} enhanced endogenous PPAR activity significantly. By contrast, the corresponding downstream metabolites, i.e., 13,14-dihydro-15-keto PGE₂ and 13,14-dihydro-15-keto PGF_{2α}, failed to increase PPAR activity.

A luciferase reporter gene was transfected to 3T3-L1 cells together with the ligand-binding domain of PPAR-alpha, PPAR-gamma or PPAR-delta fused to a yeast GAL4 DNA-binding domain. 15-keto PGE₂ and 15-keto PGF_{2α} activated PPAR-gamma and, to a lesser degree, PPAR-alpha.

Also examined was the ability of 15-keto PGE₂ to induce protein expression of adipogenesis-specific, PPAR-gamma target genes, i.e., IRS-1 and -2. Substantial amounts of PPAR-gamma1 and PPAR-gamma2 protein were detected in 3T3-L1 cells when they were treated with insulin and dexamethasone, but not methylisobutylxanthine (MIX) alone. Addition of 15-keto PGE₂ and MIX with insulin and dexamethasone significantly enhanced PPAR-gamma1 and PPAR-gamma2 expression. 15-keto PGE₂ and BRL49653 strongly induced expression of aP2, an adipocyte-specifc marker, even in the absence of MIX. In the presence of insulin and dexamethasone, BRL49653 treatment dramatically increased IRS-2 expression. 15-keto PGE₂ enhanced the expression to a level similar to MIX. Either insulin/dexamethasone or MIX induced IRS-1 expression. PPAR-gamma ligands including 15-keto PGE₂ and BRL49653 did not increase the amount of IRS-1 protein.

### 15-keto prostaglandin-Δ¹³-reductase inhibitors

Recombinant human PGR/LTB4DH and PGR2/ZADH1 proteins were expressed and their enzymatic activities examined. Similar to mouse PGR-2, all three recombinant human enzymes had 15-keto-prostaglandin-Δ¹³-reductase activity and catalyzed conversion of 15-keto prostaglandin into 13,14-dihydro-15-keto prostaglandins.

A series of compounds were tested for their inhibitory effects on 15-keto prostaglandin-Δ¹³-reductase activity. The inhibition assay was performed following the procedure described above. Different concentrations of 15-keto prostaglandin-Δ¹³-reductase inhibitors were added to the reaction mixture and incubated for 2 hours at 37°C.

15-keto prostagiandin-Δ¹³-reductase activity was found to be inhibited by the following compounds: 3,3'-methylene-bis (4-hydroxycoumarin), 4-hydroxycoumarin, warfarin, quercetin dehydrate, luteolin, kaempferol, 5,7-dihydroxyflavone, 3,3'-dihydroxyflavone, 2,2'-dihydroxychalcone, 3,2'-dihydroxychalcone, 4,2'-dihydroxychalcone, 2',4'-dihydroxychalcone, 5-bromo-2,2'-dihydroxy-4',6'-dimethoxychalcone, 2,2',5'-trihydroxychalcone, and 2,2',4'-trihydroxychalcone.

The effect of one of the 15-keto prostaglandin-Δ¹³-reductase inhibitors, i.e., 3,3'-methylene-bis (4-hydroxycoumarin), on insulin sensitivity was examined as follows. 3T3-L1 cells were induced to differentiate in the same manner as described above. A glucose transport assay was performed by measuring uptake of 2-deoxy-D-[3H]glucose as described by Fingar et al. See Fingar et al., Endocrinology 134:728-735. 1.67 µM of the inhibitor or 45 nM of a PPAR agonist, i.e., AVANDIA, was added to the cells. 10 µM cytochalasin-B was used to measure background glucose uptake levels. 100 nM of insulin was used to stimulate glucose uptake. Insulin treatment alone in differentiated 3T3-L1 cells increased glucose uptake by 20 folds. In the presence of this 15-keto prostagiandin-Δ¹³-reductase inhibitor, glucose uptake was increased by 30 folds. Of note, the ability of this inhibitor to enhance glucose uptake was comparable to that of AVANDIA.

The in vivo effect of another inhibitor, 2,2'-dihydroxychalcone, on glucose metabolism was also investigated. Female diabetic mice were obtained from the Jackson Laboratory (C57BLKS/J-m+/+*Lep*^{db}, 12 to 13 weeks old). These mice were characterized as insulin-resistant and hyperglycemic. 25 mg/kg of 2,2'-dihydroxychalcone (n=8) or placebo (n=4, final concentration against body fluid: 0.5% DMSO/1% ethanol) was injected intraperitoneally to the mice twice a day for two days. The mice were then fasted from the 48^{th} hour through 52^{nd} hour after the first injection. Blood samples were collected from the retro-orbital sinus before the first injection, and every two hours after two days of treatment. Blood glucose levels were measured by an electrode-type blood glucose meter (HORIBA, AntSense II, Japan). The inhibitor treatment induced a significant reduction of blood glucose levels, compared to the placebo controls.

An RNA interference (RNAi) approach was used to silence PGR-2 expression. Two small interfering RNA (siRNA) duplexes, i.e., gaguucaguuuaccggaug (SEQ ID NO:7) and guucaagugaggacucuuu (SEQ ID NO:8), were annealed first and then introduced into 3T3-L 1 fibroblasts or differentiating pre-adipocytes by transfection using oligofectamine (Invitrogen). Transfection of the siRNA duplexes reduced PGR-2 expression. In another experiment, transfection of the siRNA duplexes increased transcriptional activation of PPAR-gamma. Thus, one can modulate PPAR-gamma activity via silencing PGR-2 expression by RNA interference.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the claims.

## Claims

1. A method of treating a peroxisome proliferator-activated receptors (PPAR) related disease, comprising administering to a subject in need thereof an effective amount of a modulator of 15-keto prostaglandin-Δ¹³-reductase.

2. The method of claim 1, wherein the PPAR related disease is type II diabetes, obesity, dyslipidemia, coronary heart disease, inflammatory disease, or cancer.

3. The method of claim 2, wherein the PPAR related disease is type II diabetes, obesity, dyslipidemia, or coronary heart disease.

4. The method of claim 3, wherein the PPAR related disease is type II diabetes.

5. The method of claim 4, wherein the modulator is a 15-keto prostaglandin-Δ¹³-reductase inhibitor.

6. The method of claim 5, wherein the inhibitor is a coumarin, a flavonoid, or a hydroxychalcone.

7. The method of claim 3, wherein the modulator is a 15-keto prostaglandin-Δ¹³-reductase inhibitor.

8. The method of claim 7, wherein the inhibitor is a coumarin, a flavonoid, or a hydroxychalcone.

9. The method of claim 2, wherein the modulator is a 15-keto prostagiandin-Δ¹³-reductase inhibitor.

10. The method of claim 9, wherein the inhibitor is a coumarin, a flavonoid, or a hydroxychalcone.

11. A method of lowering blood glucose levels in a subject, comprising administering to the subject an effective amount of an inhibitor of 15-keto prostaglandin-Δ¹³-reductase.

12. The method of claim 11, wherein the 15-keto prostaglandin-Δ¹³-reductase is PGR/LTB4DH.

13. The method of claim 11, wherein the 15-keto prostaglandin-Δ¹³-reductase is PGR2/ZADH1.

14. The method of claim 11, wherein the inhibitor is a coumarin, a flavonoid, or a hydroxychalcone.

15. A method of identifying a compound for inhibiting activity of 15-keto prostaglandin-Δ¹³-reductase, comprising providing a system containing 15-keto prostaglandin-Δ¹³-reductase, contacting a compound with the system, determining the activity of the 15-keto prostaglandin-Δ¹³-reductase, and comparing the activity with that obtained in the same manner except that the compound is absent.

16. The method of claim 15, wherein the system is a cell containing a gene that expresses 15-keto prostaglandin-Δ¹³-reductase and the activity is determined by measuring expression activity of the gene.

17. The method of claim 15, wherein the system is a cell-free solution containing 15-keto prostaglandin-Δ¹³-reductase and the activity is determined by measuring enzymatic activity of 15-keto prostaglandin-Δ¹³-reductase.

18. The method of claim 16, wherein the 15-keto prostaglandin-Δ¹³-reductase is PGR/LTB4DH.

19. The method of claim 16, wherein the 15-keto prostaglandin-Δ¹³-reductase is PGR2/ZADH 1.

20. The method of claim 17, wherein the 15-keto prostaglandin-Δ¹³-reductase is PGR/LTB4DH.

21. The method of claim 17, wherein the 15-keto prostaglandin-Δ¹³-reductase is PGR2/ZADH1.
